# EUROPÄISCHE PATENTSCHRIFT

(11) **EP 3 706 666 B1**
(45) Veröffentlichungstag und Bekanntmachung des Hinweises auf die Patenterteilung: **16.09.2026**
(21) Anmeldenummer: 18789587.5
(22) Anmeldetag: 16.10.2018
(51) Int. Cl.: A61F 2/68, A61F 2/72, A61F 2/80, A61F 5/01, A61B 5/00

(54) **ORTHOPÄDIETECHNISCHE EINRICHTUNG**
ORTHOPEDIC DEVICE
APPAREIL ORTHOPÉDIQUE

(30) Priorität: 10.11.2017 DE 102017126463
(43) Veröffentlichungstag der Anmeldung: 16.09.2020
(73) Patentinhaber: Ottobock SE & Co. KGaA, 37115 Duderstadt (DE)
(72) Erfinder: KOPPE, Mario, 99988 Wendehausen (DE); GLINDEMANN, Heiko, 37085 Göttingen (DE); GRAIMANN, Bernhard, 37434 Obernfeld (DE)
(74) Vertreter: Gramm, Lins & Partner Patent- und Rechtsanwälte PartGmbB
(86) Internationale Anmeldenummer: PCT/EP2018/078243
(87) Internationale Veröffentlichungsnummer: WO 2019/091716

(56) Entgegenhaltungen:
- WO-A1-2014/090927
- DE-A1- 102007 035 409
- DE-A1- 102011 101 583
- DE-A1- 102013 225 702
- DE-B4- 102007 035 409
- US-A- 4 243 051
- US-A1- 2010 262 052
- US-B1- 8 591 599

## Beschreibung

Die Erfindung betrifft eine orthopädietechnische Einrichtung mit einem Grundkörper zum Anlegen an ein Körperteil des Trägers, wobei die Einrichtung wenigstens eine Elektrode zum transkutanen Übertragen elektrischer Signale aufweist. Die Erfindung betrifft zudem eine Elektrode für eine derartige orthopädietechnische Einrichtung sowie ein Verfahren zum Herstellen einer solchen Elektrode.

Derartige Elektroden werden für unterschiedliche Anwendungen verwendet. Sie werden beispielsweise bei der Muskelstimulation, beispielsweise zum Muskelaufbau durch funktionelle Elektrostimulationen (FES) oder transkutane elektrische Nervenstimulation (TENS), eingesetzt.

Dabei werden elektrische Signale über die Elektrode auf den Körper des Trägers der orthopädietechnischen Einrichtung übertragen, sodass die Muskeln zur Bewegung angeregt werden. Alternativ dazu ist es auch möglich, mit derartigen Elektroden elektrische Signale von einem Muskel oder der Haut des Trägers abzunehmen und der weiteren Verarbeitung zuzuführen. Diese Signale können zur Information über den Gesundheitszustand, den Bewegungszustand oder sonstige Parameter des Trägers herangezogen werden oder der Steuerung beispielsweise einer Orthese oder Prothese dienen. Vorzugsweise werden Biosignale wie beispielsweise myoelektrische Signale oder Impedanz-Signale erfasst.

Um die Übertragung der elektrischen Signale von der Elektrode und der orthopädietechnischen Einrichtung auf den Körper des Patienten möglich zu machen, ist es nötig, dass der tatsächliche Kontakt zwischen der Elektrode und der Haut des Patienten über ein ionisch leitendes Medium erfolgt. Leitung elektrischer Signale von der Haut des Patienten ist auch über metallische Elektroden möglich. So ist es beispielsweise aus der DE 10 2009 013 470 A1 bekannt, ein Gelkissen mit einem ionisch leitfähigen Liquidgel auf der der Haut zugewandten Seite der Elektrode anzuordnen. Auf der gegenüberliegenden Seite des Gelkissens befindet sich ein flüssigkeitsdichtes Trägermaterial, um ein Austreten des Liquidgels auf dieser Seite der Elektrode zu verhindern. Um einen möglichst guten elektrischen Kontakt zwischen der Elektrode, insbesondere dem Liquidgel im Gelkissen, und der Haut des Trägers zu gewährleisten, muss auf der Haut des Patienten ein Gel- oder Feuchtigkeitsfilm gebildet werden. Nachteilig ist, dass die mit einem Hydro- oder Liquidgel versehenen Elektroden klebrig sind, sodass sie nur kurzfristig für eine gute Haftung an der Haut des Trägers sorgen. Schwammelektroden hingegen werden durch die einfach herausdrückbare Flüssigkeit feucht und schmierig.

Eine ähnliche Elektrode ist aus EP 1 021 986 A2 bekannt. Auch hier befindet sich an der der Haut zugewandten Seite eines Trägermaterials ein Gelkörper, der mit einem elektrisch leitfähigen Gel gefüllt ist. Aus der EP 0 467 966 B1 ist eine elektrische Reizelektrode bekannt, bei der die elektrischen Signale über ein leifähiges Tuch direkt in die Haut des Patienten eingekoppelt werden.

Für metallische Elektroden werden häufig Silbertextile oder silberbeschichtete Textilien verwendet. Diese haben eine antibakterielle Wirkung und verfügen über eine ausreichende elektrische Leitfähigkeit. Nachteilig ist jedoch, dass sie insbesondere bei langem Tragen zu Hautreizungen führen können und zudem insbesondere durch vom Träger abgesonderten Schweiß korrodiert werden können. Über einen langen Tragezeitraum verlieren die Elektroden die elektrische Leitfähigkeit, sodass insbesondere die Qualität abgenommener Signale nachlässt und diese ab einem gewissen Zeitpunkt nicht mehr zu Steuerung von Prothesen verwendet werden können.

Aus der DE 10 2011 101 583 A1 ist eine Elektrode bekannt, bei der eine Schicht, die vorzugsweise ein Vlies ist, teilweise in einer elektrisch leitfähigen Polymerschicht eingebracht ist, sodass die Vliesschicht auf einer Seite aus der Polymerschicht heraussteht. Die Vliesschicht ist ausgebildet, eine Flüssigkeit zumindest auch auf Grund der Kapillarkraft festzuhalten, sodass eine leitfähige Flüssigkeit leicht festgehalten werden kann, ohne dass es zu austretendem Gel und den damit verbundenen Nachteilen kommt. Nachteilig ist jedoch auch hier, dass die Elektrode relativ aufwendig herzustellen ist, sodass die Produktionskosten relativ hoch sind.

Aus der DE 10 2013 225 702 A1 ist eine textilbasierte Elektrode bekannt, die ein leitfähiges Textil mit elektrisch leitenden Fäden und ein magnetisierbares Polymer aufweist. Dabei wird zunächst das Textil mit den elektrisch leitfähigen Fäden und das magnetisierbare Polymer separat voneinander hergestellt. Anschließend werden diese zur Elektrode zusammengefügt und das Polymer magnetisiert.

Die US 2010/0262052 A1 beschreibt eine Gelenks Orthese, mit der Arthritis behandelt werden soll. Dazu verfügt die Orthese über Elektroden zur Elektrostimulation.

Der Erfindung liegt die Aufgabe zugrunde, eine orthopädietechnische Einrichtung so weiter zu entwickeln, dass sie einfach und kostengünstig herstellbar ist und ein Kontakt zwischen der Elektrode und dem Grundmaterial eines Grundkörpers der orthopädietechnischen Einrichtung leicht und zuverlässig herstellbar ist.

Eine orthopädietechnische Einrichtung ist beispielsweise ein Prothesenschaft oder ein Prothesenliner beispielsweise für eine Armprothese oder eine Beinprothese, wobei weitere Elemente und Bauteile der Prothese an dem Prothesenschaft oder dem Prothesenliner angeordnet sein können. Eine orthopädietechnische Einrichtung ist beispielsweise eine Orthese für die obere oder untere Extremität oder eine Bandage. Eine orthopädietechnische Einrichtung kann eine Steuereinrichtung, insbesondere mit einer elektronischen Datenverarbeitungseinrichtung, aufweisen, um beispielsweise Biosignale zu verarbeiten und/oder Stimulationssignale an den Körper und die Hautoberfläche zu senden. Üblicherweise weist die Steuereinrichtung einen Mikroprozessor. Die Steuereinrichtung kann mit wenigstens einem Sensor, vorzugsweise mit mehreren Sensoren gekoppelt sein, die Teil der orthopädietechnischen Einrichtung sein können. Durch den Sensor oder die Sensoren können beispielsweise Aktionszustände ermittelt werden. Es können kinetische Sensoren, beispielsweise Kraft- oder Momentensensoren, und/oder kinematische Sensoren, beispielsweise Beschleunigungs-, Geschwindigkeits- oder Lagesensoren, und/oder Umgebungssensoren, beispielsweise Ultraschall oder Radar, verwendet werden.

Die Erfindung löst die gestellte Aufgabe durch eine orthopädietechnische Einrichtung mit einem Grundkörper zum Anlegen an ein Körperteil des Trägers, wobei die Einreichung wenigstens eine Elektrode zum transkutanen Übertragen elektrische Signale aufweist, wobei die wenigstens eine Elektrode ein elektrisch leitfähiges Textil und ein elektrisch leitfähiges Polymer aufweist, wobei das elektrisch leitfähige Textil teilweise von dem elektrisch leitfähigen Polymer durchdrungen wird und derart angeordnet ist, dass es mit dem Körperteil in Kontakt kommt, wenn der Grundkörper angelegt ist.

Das elektrisch leitfähige Textil ist teilweise von dem elektrisch leitfähigen Polymer durchdrungen. Dies bedeutet, dass das leitfähige Textil auf einer Seite aus dem leitfähigen Polymer herausragt. Besonders bevorzugt wird dadurch erreicht, , dass das elektrisch leitfähige Polymer nicht mit dem Körper in Kontakt kommt, wenn der Grundkörper der orthopädietechnischen Einrichtung am Körperteil des Trägers angelegt ist. Lediglich das elektrisch leitfähige Textil kommt in diesem Fall in Kontakt. Dadurch wird erreicht, dass elektrische Signale vom Körperteil des Trägers besonders gut und stabil abgenommen werden können. Durch das elektrisch leitfähige Polymer, dass das elektrisch leitfähige Textil teilweise durchdringt, werden die einzelnen Fasern des Textils in Position gehalten, wodurch Artefakte im Signal durch intertextile Bewegungen stark reduziert oder vollständig eliminiert werden können. Der sehr gute mechanische Kontakt zwischen dem leitfähigen Polymer und dem leitfähigen Textil sorgt dafür, dass ein großflächiger elektrischer Kontakt zwischen den beiden Materialien herrscht, was für einen robusten Aufbau sorgt.

Es ist jedoch auch möglich, dass das elektrisch leitfähige Polymer das Textil so weit durchdrungen hat, dass es zumindest in einem Bereich mit der Haut des Trägers in Kontakt kommt. Alternativ oder zusätzlich kann das Textil mit einem Loch oder einer Öffnung ausgerüstet sein, so dass das Polymer im Bereich des Loches oder der Öffnung mit der Haut des Trägers in Kontakt kommt.

Aus dem Stand der Technik bekannte Elektroden aus einem elektrisch leitfähigen Polymer sind anfällig für Artefakte und ungewollte Störungen im Messsignal, die wahrscheinlich durch die glatte hydrophobe Oberfläche der aus diesem Material hergestellten Elektrode begründet werden kann. Durch den guten Kontakt zwischen dem leitfähigen Textil und dem leitfähigen Polymer wird auch dieser Nachteil stark reduziert oder vollständig ausgeschaltet.

Hinzu, dass anders als im Stand der Technik, in dem ein Flies teilweise im elektrisch leitfähigen Polymer enthalten ist und aus diesem hervorsteht, keine Gele oder eine sonstige Flüssigkeit verwendet werden muss, um einen guten Kontakt zwischen der Haut des Trägers und der Elektrode herzustellen. Vielmehr wird dies automatisch durch das elektrisch leitfähige Textil und das dieses teilweise durchdringende elektrisch leifähige Polymer gewährleistet.

Vorteilhafterweise ist das leitfähige Textil ein Silbertextil oder ein silberbeschichtetes Textil. Neben der antibakteriellen Wirkung des Silbers hat diese Ausführungsform den Vorteil, dass das Textil hydrophil ist und somit automatisch eine leitfähige Flüssigkeitsschicht bildet, die zu einem guten elektrischen Kontakt zwischen dem elektrisch leitfähigen Textil und der Haut des Trägers führt.

Vorzugsweise ist das elektrisch leitfähige Polymer ein Silikon, ein Polyurethan oder ein thermoplastisches Polymerjeweils mit elektrisch leitfähigen Partikeln. Dieses Silikon ist hydrophob und sorgt auf diese Weise für eine starke Beschränkung der an dem hydrophilen Textil angelagerten Flüssigkeit, sodass eine Korrosion des Silbers minimiert werden kann. Dadurch werden die Lebensdauer der entsprechenden Elektroden und damit die Lebensdauer der orthopädietechnischen Einrichtung vergrößert und die Gefahr von Hautreizungen verkleinert.

Die elektrisch leitfähigen Partikel in dem elektrisch leitfähigen Polymer sind vorteilhafterweise Metallpartikel, insbesondere Silberpartikel, oder Kohlenstoffpartikel, die vorzugsweise in Form von Rußpartikeln, Graphitpartikeln oder Kohlenstoffnanoröhrchen vorliegen.

In einer bevorzugten Ausgestaltung der orthopädietechnischen Einrichtung ist der Grundkörper aus einem Grundkörpermaterial hergestellt und die wenigstens eine Elektrode mit dem Grundkörpermaterial verbunden, insbesondere vergossen oder darin eingegossen, und/oder in ein Textil eingearbeitet. Dies ist insbesondere dann der Fall, wenn es sich bei der orthopädietechnischen Einrichtung um einen Prothesenliner oder ein sonstiges Bauteil insbesondere aus einem Polymer, besonders vorzugsweise aus einem Silikon, handelt. Auf diese Weise ist ein guter mechanischer Kontakt zwischen dem Grundkörpermaterial des Grundkörpers und der Elektrode gewährleistet, sodass einerseits das Herstellungsverfahren vereinfacht und damit kostengünstiger ausgebildet werden kann und andererseits die Gefahr von sich vom Grundkörper lösenden Elektroden reduziert wird, wodurch wieder die Lebensdauer der orthopädietechnischen Einrichtung erhöht und die Gefahr von Hautreizungen durch schadhafte orthopädietechnische Einrichtungen verringert wird.

Vorteilhafterweise ist an einer dem Textil abgewandten Seite des Polymers ein Befestigungselement, vorzugsweise ein Formschlusselement, angeordnet, mit dem die Elektrode an dem Grundkörper befestigt oder befestigbar ist. Das Formschlusselement kann vorteilhafterweise ein Druckkopf oder eine Schraube sein. In diesem Fall ist es von Vorteil, wenn sich am Grundkörper ein korrespondierend ausgebildetes zweites Formschlusselement befindet, dass mit dem Formschlusselement der Elektrode zusammenwirkt. Insbesondere bei der Verwendung lösbarer Formschlusselemente, wie beispielsweise einem Druckkopf, einer Schnappverbindung oder einem Schraubgewinde, das in ein entsprechendes Gegengewinde eingeschraubt wird, lassen sich Elektroden leicht austauschen oder für Reinigung und/oder Wartung entfernen.

Vorteilhafterweise ist die wenigstens eine Elektrode mit einem elektrischen Leiter verbunden, der einen Kern aus einem elektrisch leitfähigen Polymer und eine elektrisch isolierende Beschichtung aufweist. Dabei hat es sich als vorteilhaft herausgestellt, wenn das elektrisch leitfähige Polymer der Elektrode und das elektrisch leitfähige Polymer des Kerns gleich sind. Auf diese Weise wird auf besonders einfache Weise gewährleistet, dass ein guter elektrischer Kontakt zwischen der Elektrode und dem elektrischen Leiter erreicht werden kann. Die Verwendung eines elektrisch leitfähigen Polymers als Kern des elektrischen Leiters sorgt dafür, dass auch der Leiter elastische und flexible Eigenschaften hat, die vorzugsweise den Elastizitätseigenschaften des Grundkörpers der orthopädietechnischen Einrichtung, insbesondere eines Prothesenliners, nicht nachstehen. Daher können auch starke mechanische Belastungen auch über einen langen Zeitraum hinweg die Funktionsfähigkeit nicht beeinträchtigen.

Das elektrisch leitfähige Textil verbindet vorzugsweise die Elektrode mit dem Material des Grundkörpers der orthopädietechnischen Einrichtung, beispielsweise des Liners. Dabei kann das Textil die einzige Verbindung oder nur eine von mehreren Verbindungen zwischen der Elektrode und dem Grundkörpermaterial sein. Die zusätzliche Verbindung zwischen der Elektrode und dem Grundkörpermaterial ist insbesondere dann von Vorteil, wenn das Grundkörpermaterial ein Elastomer ist, das mit dem elektrisch leitfähigen Polymer nicht oder nur sehr schlecht verbindbar ist. Auf diese Weise wird dennoch eine ausreichende mechanische Verbindung erreicht. Vorzugsweise ist das elektrisch leitfähige Textil so ausgebildet, dass es sich über die gesamte Kontaktfläche der Elektrode erstreckt, die mit dem Körperteil in Kontakt kommen soll. Dies ist jedoch nicht notwendigerweise der Fall. Für bestimmte Ausführungsformen ist es vorteilhaft, wenn das elektrisch leitfähige Textil ringförmig ausgebildet ist, wobei damit nicht ausschließlich, aber auch kreisförmige Ausgestaltungen gemeint sind. Unter ringförmig wird vorliegend jede Ausgestaltung der geometrischen Form des elektrisch leitfähigen Textils verstanden, die ein Loch aufweist. Durch dieses hindurch wird vorteilhafterweise das elektrisch leitfähige Polymer mit dem Körperteil des Patienten in Kontakt gebracht, wenn der Grundkörper angelegt wird. Das ringförmige elektrisch leitfähige Textil kommt in diesem Fall zwar auch mit dem Körperteil in Kontakt, dient jedoch zu einem Großteil auch der Verbindung der Elektrode mit dem Grundkörpermaterial.

Die Erfindung löst die gestellte Aufgabe zudem durch eine Elektrode für eine hier beschriebene orthopädietechnische Einrichtung. Zudem löst sie die gestellte Aufgabe durch ein Verfahren zum Herstellen einer solchen Elektrode, dass sich dadurch auszeichnet, dass das elektrisch leitfähige Polymer im teilweise oder vollständig ausgehärteten Zustand und das elektrisch leitfähige Textil übereinander gelegt werden und durch Druck soweit miteinander verpresst werden, dass das Polymer das Textil teilweise, vorzugsweise zur Hälfte, durchdringt.

Mit Hilfe der beigefügten Figur wird nachfolgend ein Ausführungsbeispiel der vorliegenden Erfindung näher erläutert. Es zeigt
- Figur 1 -: die Draufsicht und die Schnittdarstellung einer Elektrode in einem Ausschnitt eines Grundkörpers einer orthopädietechnischen Einrichtung,
- Figur 2 - 6 -: schematische Darstellungen unterschiedlicher orthopädietechnischer Einrichtungen gemäß Ausführungsbeispielen der vorliegenden Erfindung,
- Figur 7 -: schematische Schnittdarstellungen durch eingebettete Elektroden,
- Figur 8 -: schematische Draufsichten auf Elektroden und
- Figuren 9 und 10 -: schematische Schnittdarstellungen durch Elektroden.

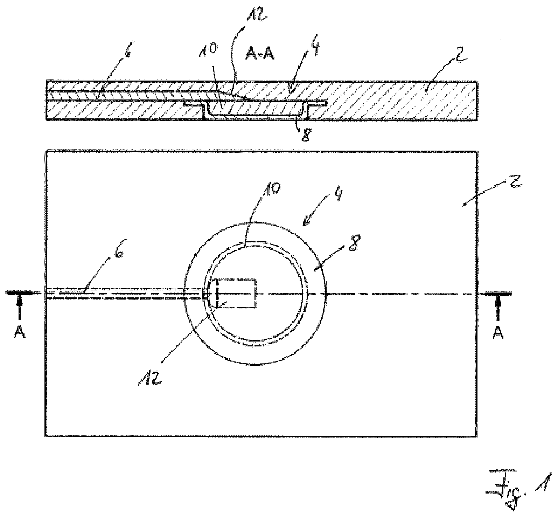

Figur 1 zeigt im unteren Bereich eine Draufsicht auf einen Ausschnitt eines Grundkörpers 2 einer orthopädietechnischen Einrichtung gemäß einem ersten Ausführungsbeispiel der vorliegenden Erfindung. Er besteht beispielsweise aus einem Silikon und verfügt über eine Elektrode 4, die mit einem elektrischen Leiter 6 verbunden ist. Figur 1 zeigt eine Draufsicht auf eine dem Körperteil des Trägers zugewandte Seite. Die Elektrode 4 verfügt über ein leitfähiges Textil 8 das mit dem Körper des Trägers in Kontakt kommt, sobald der Grundkörper 2 an das Körperteil des Trägers angeordnet ist. Das leitfähige Textil 8 wird von einem leitfähigen Polymer 10 teilweise durchdrungen, sodass ein guter mechanischer und elektrischer Kontakt zwischen dem leitfähigen Textil 8 und dem leitfähigen Polymer 10 hergestellt ist. In einem Kontaktbereich 12 ist der elektrische Leiter 6 mit der Elektrode 4 verbunden.

Im oberen Teil der Figur 1 ist ein Querschnitt entlang der Linie A-A dargestellt. Man erkennt den Grundkörper 2, in dem sich der elektrische Leiter 6 und die Elektrode 4 befinden. Das elektrisch leitfähige Textil 8 ist topfförmig ausgebildet. Darin befindet sich leitfähige Polymer 10, das in einem Kontaktbereich 12, in dem der Leiter 6 abgeschrägt ist, mit dem elektrischen Leiter 6 in Kontakt kommt. In Figur 1 nicht dargestellt ist das teilweise Durchdringen des leitfähigen Textils durch das leitfähige Polymer.

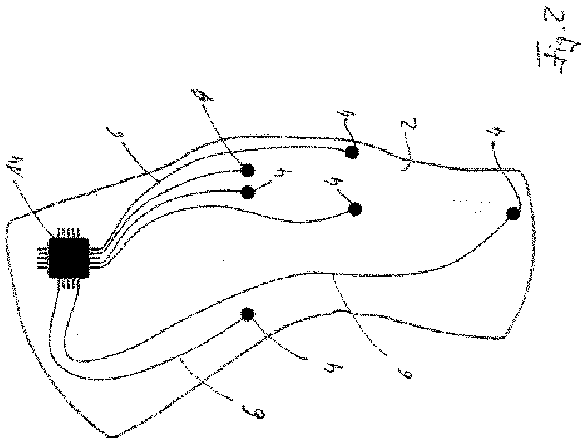

Figur 2 zeigt eine orthopädietechnische Einrichtung in Form einer Kniebandage. Sie verfügt über einen Grundkörper 2, der beispielsweise aus einem elastischen Textil hergestellt sein kann und über Verdickungen, Pelotten oder eingeschobene oder eingearbeitete Polsterelemente verfügen kann. Im gezeigten Ausführungsbeispiel sind in den Grundkörper 2 sechs Elektroden 4 eingearbeitet, die jeweils mit einem elektrischen Leiter 6 verbunden sind. Die elektrischen Leiter 6 verbinden die Elektroden 4 mit einer elektrischen Steuerung 14, die in Figur 2 schematisch dargestellt ist. Die elektrische Steuerung 14 ist eingerichtet, elektrische Signale, die von den Elektroden 4 über die elektrischen Leiter 6 an die elektrische Steuerung 14 gesendet werden, weiter zu verarbeiten und gegebenenfalls an eine elektronische Datenverarbeitungseinrichtung zu übermitteln. Dies kann beispielsweise über in Figur 2 nicht dargestellte Kabel geschehen, was insbesondere dann von Vorteil ist, wenn die elektronische Datenverarbeitungseinrichtung in der elektrischen Steuerung 14 oder zumindest am Grundkörper 2 der orthopädietechnischen Einrichtung angeordnet ist. So kann es beispielsweise sinnvoll sein, die von den Elektroden 4 übermittelten elektrischen Signale in Form von elektronischen Daten in der elektrischen Steuerung 14 so weiterzuverarbeiten, dass sie in einem elektronischen Datenspeicher, der vorteilhafterweise Teil der orthopädietechnischen Einrichtung ist, hinterlegt werden können, bis sie ausgelesen und ausgewertet werden können. Alternativ oder zusätzlich dazu ist es von Vorteil, eine drahtlose Übermittlung der elektronischen Daten von der elektrischen Steuerung 14 auf eine elektronische Datenverarbeitungseinrichtung zu ermöglichen.

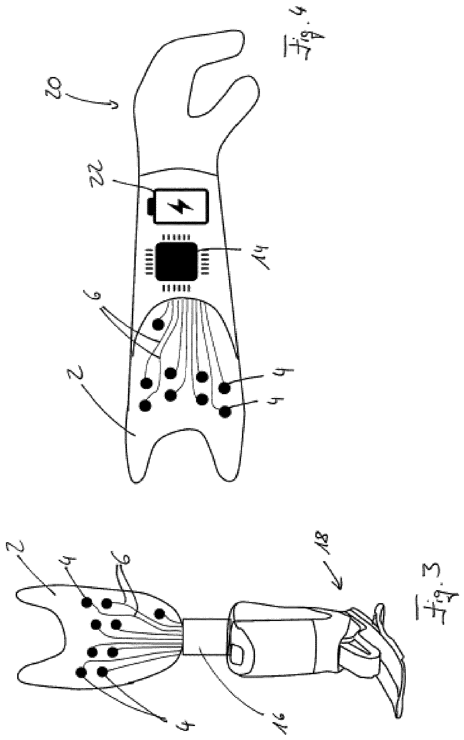

Figur 3 zeigt eine orthopädietechnische Einrichtung gemäß einem weiteren Ausführungsbeispiel der vorliegenden Erfindung in Form einer Unterschenkelprothese. Der Grundkörper 2 ist in Form des Prothesenschaftes ausgebildet, an dem die Elektroden 4 angeordnet und über elektrische Leiter 6 mit einer elektrischen Steuerung verbunden sind, die nicht dargestellt ist. Am Grundkörper 2 befindet sich ein Unterschenkelelement 16 sowie ein künstlicher Fuß 18. Über die Elektroden 6 können beispielsweise myoelektrische Signale von einem Amputationsstumpf abgenommen werden, der in dem Grundkörper 2 angeordnet wird. Diese myoelektrischen Signale werden über die elektrischen Leiter 6 zur elektrischen Steuerung geleitet und zur Steuerung des künstlichen Fußes 18 verwendet.

Figur 4 zeigt eine weitere Ausführungsform einer orthopädietechnischen Einrichtung gemäß einem Ausführungsbeispiel der vorliegenden Erfindung, die ebenfalls als Prothese, nun jedoch als Handprothese und Unterarmprothese ausgebildet ist. Auch hier bildet der Grundkörper 2 den Prothesenschaft, an dem die Elektroden 4 angeordnet und über elektrische Leiter 6 mit der elektrischen Steuerung 14 verbunden sind. Die elektrische Steuerung 14 ist eingerichtet, aus den von den Elektroden 4 abgenommenen elektrischen Signalen Steuersignale für eine Prothesenhand 20 zu generieren und so Funktionen der Prothesenhand 20 zu steuern. Die in Figur 4 gezeigte orthopädietechnische Einrichtung verfügt zudem über eine Stromversorgung 22, durch die die elektrische Steuerung 14 mit elektrischem Strom versorgbar ist.

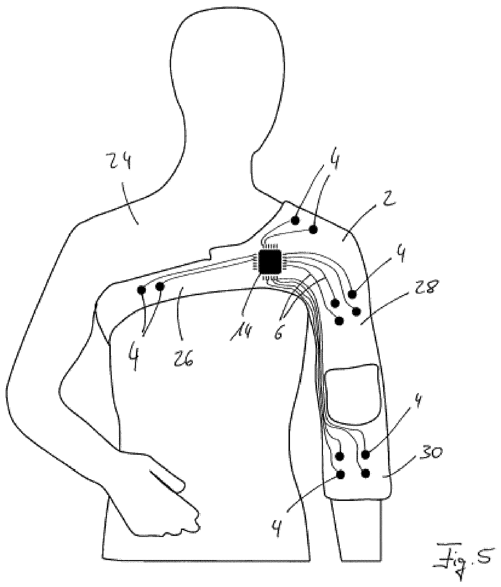

Figur 5 zeigt eine Ausführungsform einer orthopädietechnischen Einrichtung, die als Schulterorthese ausgebildet ist. Der Grundkörper 2 erstreckt sich über den Schulterbereich des Trägers 24 und entlang des Arms des Trägers 24 bis über den Ellbogen hinaus. Der Grundkörper 2 verfügt über einen Befestigungsgurt 26, der um den Rumpf des Trägers herumgeführt wird. Sowohl an dem Befestigungsgurt 26, der Teil des Grundkörpers 2 ist, als auch an anderen Teilen des Grundkörpers 2, beispielsweise einem Oberarmelement 28 und einem Unterarmelement 30 sind Elektroden 4 angeordnet, die durch elektrische Leiter 6 mit der elektrischen Steuerung 14 verbunden sind.

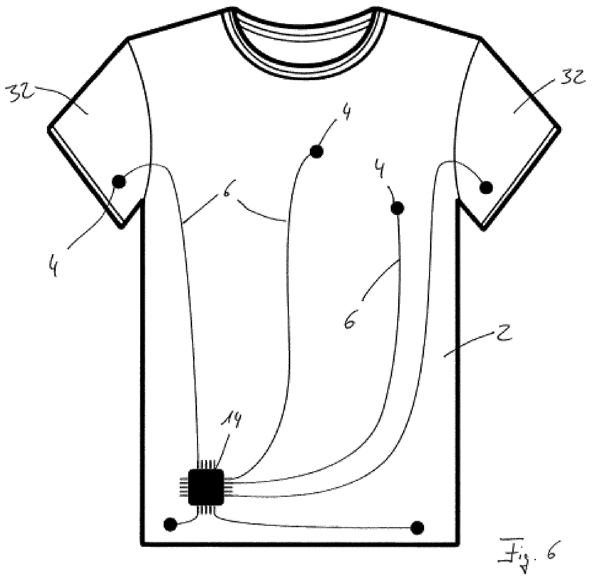

Figur 6 zeigt eine orthopädietechnische Einrichtung in Form eines T-Shirts, wobei der Grundkörper 2 das T-Shirt selber bildet. Sowohl an den Ärmeln 32 als auch am Rest des Grundkörpers 2 sind Elektroden 4 angeordnet, die wieder über elektrische Leiter 6 mit der elektrischen Steuerung 14 verbunden sind. Die Schwierigkeit bei dieser Ausgestaltung der orthopädietechnischen Einrichtung besteht darin, einen möglichst guten Kontakt der Elektroden zum Hautbereich des Trägers aufzubauen. Dies kann beispielsweise über den Schnitt des T-Shirts, ein elastisches Material oder eingearbeitete Gurte geschehen.

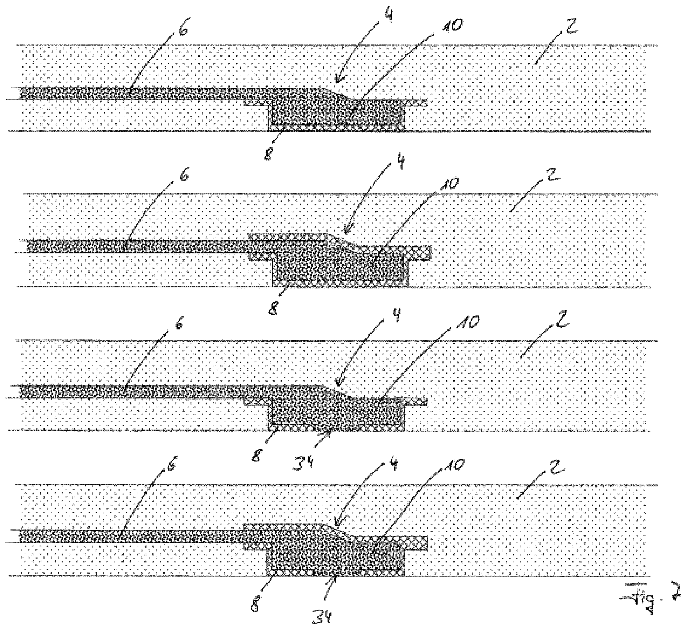

Figur 7 zeigt vier unterschiedliche Schnittdarstellung durch jeweils eine Elektrode 4, die in den Grundkörper 2 einer orthopädietechnischen Einrichtung eingebettet ist. Sie verfügt über ein leitfähiges Polymer 10, das mit einem elektrischen Leiter 6 verbunden ist. An den in Figur 7 dargestellten Unterseiten des jeweiligen Grundkörpers 2, die dem Körper des Patienten oder Trägers der orthopädietechnischen Einrichtung zugewandt ist, befindet sich das leitfähige Textil 8, das in den unteren beiden Schnittdarstellungen eine Öffnung 34 aufweist. An diesen Stellen kommt das leitfähige Polymer 10 direkt mit der Haut des Trägers in Kontakt. Bei der zweiten und vierten Darstellung von oben befindet sich das leitfähige Textil 8 nicht nur an der genannten Unterseite, sondern umschließt das leitfähige Polymer 10 an drei Seiten. Es befindet sich insbesondere auch im Kontaktbereich zwischen dem Grundkörper 2 und dem leitfähigen Polymer 10. Das leitfähige Textil 8 hat an dieser Stelle insbesondere die Aufgabe, die beiden gegebenenfalls ansonsten nicht oder nur schwer miteinander verbindbaren Polymere miteinander zu verbinden.

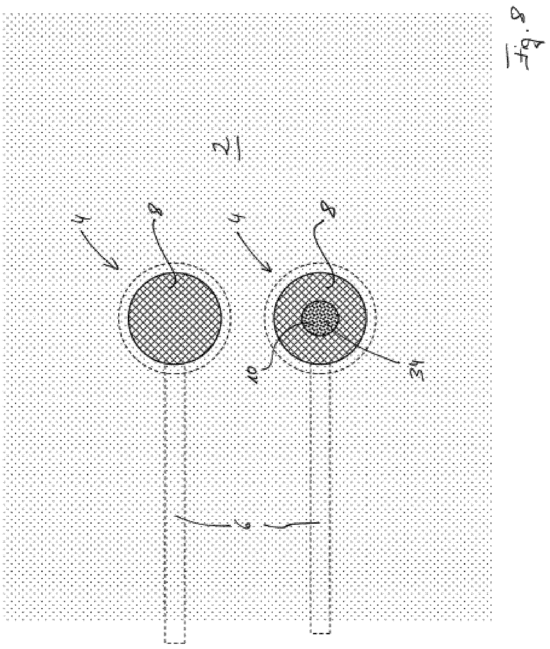

In Figur 8 sind zwei Elektroden 4 dargestellt, die unterschiedlich ausgebildete leitfähige Textile 8 aufweisen. Gezeigt ist die schematische Ansicht von der Seite des Grundkörpers 2, die mit der Haut des Trägers in Kontakt kommt. Die in Figur 8 obere gezeigte Elektrode 4 verfügt über ein vollflächiges leitfähiges Textil 8, das mit der Haut des Trägers in Kontakt kommt. Die untere Darstellung zeigt eine Elektrode 4, die über die bereits beschriebene Öffnung 34 verfügt, in der folglich das leitfähige Polymer 10 mit der Haut des Trägers in Kontakt kommt. Beide Elektroden sind über einen elektrischen Leiter 6 verbunden.

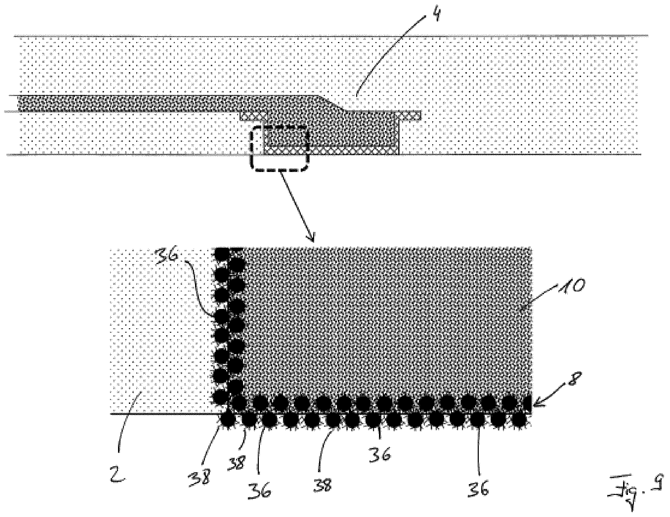

Figur 9 zeigt im oberen Bereich die schematische Schnittdarstellung durch die Elektrode 4, die der obersten Darstellung aus Figur 7 entspricht. Die untere Darstellung der Figur 9 entspricht einer detaillierteren Ansicht des eingekesselten Bereiches. Man erkennt, das Material des Grundkörpers 2, das elektrisch leitfähige Polymer 10 sowie die Faserstruktur des leitfähigen Textils 8. Gezeigt ist die Schnittdarstellung durch einzelne Fasern 36. Man erkennt, dass das leitfähige Polymer 10 etwa halb in das leitfähige Textil 8 eingedrungen ist. An den einzelnen Fasern 36 befinden sich Mikrofasern 38, die für einen guten Kontakt und eine gute Verbindung sowohl zum jeweiligen Polymer des Grundkörpers 2 oder zum leitfähigen Polymer 10 als auch zur Haut des Trägers sorgen.

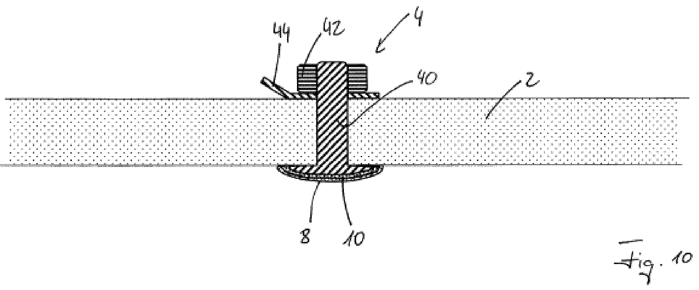

Figur 10 zeigt eine gänzlich andere Ausgestaltung der Elektrode 4. Sie verfügt über das leitfähige Textil 8, das eine Schicht aus leitfähigem Polymer 10 umgibt, das an einer Schraube 40 angeordnet ist. Diese ist durch den Grundkörper 2 der orthopädietechnischen Einrichtung hindurchgeführt und mit einer Mutter 42 gesichert. Sowohl die Schraube 40 als auch die Mutter 42 bestehen vorteilhafterweise aus Metall, um einen guten elektrischen Kontakt herzustellen. Zwischen dem Grundkörper 2 und der Mutter 42 ist ein Kabelschuh 44 angeordnet, der mit einem elektrischen Leiter 6 verbindbar ist.

### Bezugszeichenliste:

- 2: Grundkörper
- 4: Elektrode
- 6: elektrischer Leiter
- 8: leitfähiges Textil
- 10: leitfähiges Polymer
- 12: Kontaktbereich
- 14: elektrische Steuerung
- 16: Unterschenkelelement
- 18: künstlicher Fuß
- 20: Prothesenhand
- 22: Stromversorgung
- 24: Träger
- 26: Befestigungsgurt
- 28: Oberarmelement
- 30: Unterarmelement
- 32: Ärmel
- 34: Öffnung
- 36: Faser
- 38: Mikrofaser
- 40: Schraube
- 42: Mutter
- 44: Kabelschuh

## Patentansprüche

1. Elektrode (4) für eine Orthopädietechnische Einrichtung zum transkutanen Übertragen elektrischer Signale, wobei die wenigstens eine Elektrode (4)
- ein elektrisch leitfähiges Textil (8) und
- ein elektrisch leitfähiges Polymer (10) aufweist,
wobei das elektrisch leitfähige Textil (8) teilweise von dem elektrisch leitfähigen Polymer (10) durchdrungen wird und eingerichtet ist, mit der Haut eines Trägers der Elektrode in Kontakt zu kommen,
**dadurch gekennzeichnet, dass**
das Textil keine Gele und keine Flüssigkeiten aufweist.

2. Elektrode (4) nach Anspruch 1, **dadurch gekennzeichnet, dass** das leitfähige Textil (8) ein Silbertextil oder ein silberbeschichtetes Textil ist.

3. Elektrode (4) nach Anspruch 1 oder 2, **dadurch gekennzeichnet, dass** das elektrisch leitfähige Polymer (10) ein Silikon, ein Polyurethan oder ein thermoplastisches Polymer jeweils mit elektrisch leitfähigen Partikeln ist.

4. Elektrode (4) nach Anspruch 3, **dadurch gekennzeichnet, dass** die Partikel Metallpartikel, insbesondere Silber, oder Kohlenstoffpartikel, insbesondere Rußpartikel, Graphitpartikel oder Kohlenstoffnanoröhren sind.

5. Orthopädietechnische Einrichtung mit einem Grundkörper (2) zum Anlegen an ein Körperteil eines Trägers, wobei die Einrichtung wenigstens eine Elektrode (4) nach einem der vorstehenden Ansprüche aufweist, **dadurch gekennzeichnet, dass** das elektrisch leitfähige Textil (8) derart angeordnet ist, dass es mit dem Körperteil in Kontakt kommt, wenn der Grundkörper (2) angelegt ist.

6. Orthopädietechnische Einrichtung nach Anspruch 5, **dadurch gekennzeichnet, dass** der Grundkörper (2) aus einem Grundkörpermaterial hergestellt ist und die wenigstens eine Elektrode (4) mit dem Grundkörpermaterial verbunden, insbesondere vergossen oder darin eingegossen, und/oder in ein Textil eingearbeitet ist.

7. Orthopädietechnische Einrichtung nach Anspruch 6, **dadurch gekennzeichnet, dass** an einer dem Textil (8) abgewandten Seite des Polymers (10) ein Befestigungselement, vorzugsweise ein Formschlusselement, besonders bevorzugt ein Druckknopf oder eine Schraube, angeordnet ist, mit dem die Elektrode an dem Grundkörper (2) befestigt oder befestigbar ist.

8. Orthopädietechnische Einrichtung nach einem der Ansprüche 5 bis 7, **dadurch gekennzeichnet, dass** die wenigstens eine Elektrode (4) mit einem elektrischen Leiter (6) verbunden ist, der einen Kern aus einem elektrisch leitfähigen Polymer und eine elektrisch isolierende Beschichtung aufweist.

9. Orthopädietechnische Einrichtung nach Anspruch 8, **dadurch gekennzeichnet, dass** das elektrisch leitfähige Polymer (10) der Elektrode und das elektrisch leitfähige Polymer des Kerns gleich sind.

10. Verfahren zum Herstellen einer Elektrode (4) nach einem der Ansprüche 1 bis 4, **dadurch gekennzeichnet, dass** das elektrisch leitfähige Polymer (10) im teilweise oder vollständig ausgehärteten oder teilweise oder vollständig vernetzten Zustand und das elektrisch leitfähige Textil (8) übereinander gelegt werden und durch Druck soweit miteinander verpresst werden, dass das Polymer (10) das Textil (8) teilweise, vorzugsweise zur Hälfte durchdringt.

## Claims

1. An electrode (4) for an orthopedic device for the transcutaneous transmission of electrical signals, wherein the at least one electrode (4) comprises
- an electrically conductive textile (8) and
- an electrically conductive polymer (10),
wherein the electrically conductive textile (8) is partially penetrated by the electrically conductive polymer (10) and configured to come into contact with the body part **characterized in that** the textile does not comprise and gels and liquids.

2. An electrode (4) according to claim 1, **characterized by** the fact that the conductive textile (8) is a silver textile or a silver-coated textile.

3. An electrode (4) according to claim 1 or 2, **characterized by** the fact that the electrically conductive polymer (10) is a silicone, a polyurethane or a thermoplastic polymer, each with electrically conductive particles.

4. An electrode (4) according to claim 3, **characterized by** the fact that the particles are metal particles, especially silver, or carbon particles, especially soot particles, graphite particles or carbon nanotubes.

5. An orthopedic device with a base body (2) for applying to a body part of the wearer, wherein the device comprises at least one electrode (4) according to one of the preceding claims, **characterized in that** the electrically conductive textile (8) is arranged in such a way that it comes into contact with the body part when the base body (2) is applied.

6. An orthopedic device according to claim 5, **characterized by** the fact that the base body (2) is made of a base body material and the at least one electrode (4) is connected to the base body material, particularly cast or moulded in said material, and/or incorporated into a textile.

7. An orthopedic device according ot claim 6, **characterized by** the fact that a fixing element, preferably a positive-locking element, especially preferably a button or a screw, is arranged on a side of the polymer (10) facing away from the textile (8), wherein the electrode is or can be fixed to the base body (2) by means of said fixing element.

8. An orthopedic device according to one of claims 5 to 7, **characterized by** the fact that the at least one electrode (4) is connected to an electric conductor (6), which comprises a core made of an electrically conductive polymer and an electrically insulating coating.

9. An orthopedic device according to claim 8, **characterized by** the fact that the electrically conductive polymer (10) of the electrode is the same as the electrically conductive polymer of the core.

10. A method for producing an electrode (4) according to one of claims 1 to 4, **characterized by** the fact that the electrically conductive polymer (10) being either partially or completely hardened or partially or completely cross-linked, and the electronically conductive textile (8) are placed on top of one another and pressed together using pressure until the polymer (10) partially penetrates the textile (8), preferably halfway.

## Revendications

1. Électrode (4) destinée à un dispositif orthopédique pour la transmission transcutanée de signaux électriques, ladite au moins une électrode (4) comprenant
- un textile électriquement conducteur (8) et
- un polymère électriquement conducteur (10),
le textile électriquement conducteur (8) étant partiellement pénétré par le polymère électriquement conducteur (10) et étant agencé pour entrer en contact avec la peau d'un porteur de l'électrode,
**caractérisée en ce que** le textile ne contient ni gel ni liquide.

2. Électrode (4) selon la revendication 1,
**caractérisée en ce que** le textile conducteur (8) est un textile en argent ou un textile revêtu d'argent.

3. Électrode (4) selon la revendication 1 ou 2,
**caractérisée en ce que** le polymère électriquement conducteur (10) est un silicone, un polyuréthane ou un polymère thermoplastique, chacun contenant des particules électriquement conductrices.

4. Électrode (4) selon la revendication 3,
**caractérisée en ce que** les particules sont des particules métalliques, en particulier d'argent, ou des particules de carbone, en particulier des particules de suie, des particules de graphite ou des nanotubes de carbone.

5. Dispositif orthopédique comprenant un corps de base (2) destiné à être appliqué sur une partie du corps d'un porteur, le dispositif comportant au moins une électrode (4) selon l'une des revendications précédentes,
**caractérisé en ce que** le textile électriquement conducteur (8) est agencé de manière à entrer en contact avec la partie du corps lorsque le corps de base (2) est appliqué.

6. Dispositif orthopédique selon la revendication 5,
**caractérisé en ce que** le corps de base (2) est fabriqué à partir d'un matériau de corps de base et **en ce que** ladite au moins une électrode (4) est reliée au matériau de corps de base, en particulier en étant enrobée ou moulée dans celui-ci, et/ou est intégrée dans un textile.

7. Dispositif orthopédique selon la revendication 6,
**caractérisé en ce que**, sur une face du polymère (10) détournée du textile (8), il est prévu un élément de fixation, de préférence un élément de liaison par complémentarité de formes, de manière particulièrement préférée un bouton-pression ou une vis, à l'aide duquel l'électrode est fixée ou peut être fixée au corps de base (2).

8. Dispositif orthopédique selon l'une des revendications 5 à 7,
**caractérisé en ce que** ladite au moins une électrode (4) est reliée à un conducteur électrique (6) qui comporte une âme en polymère électriquement conducteur et un revêtement électriquement isolant.

9. Dispositif orthopédique selon la revendication 8,
**caractérisé en ce que** le polymère électriquement conducteur (10) de l'électrode et le polymère électriquement conducteur de l'âme sont identiques.

10. Procédé de fabrication d'une électrode (4) selon l'une des revendications 1 à 4,
**caractérisé en ce que** le polymère électriquement conducteur (10), à l'état partiellement ou totalement durci ou partiellement ou totalement réticulé, et le textile électriquement conducteur (8) sont superposés et pressés l'un contre l'autre par une pression telle que le polymère (10) pénètre partiellement, de préférence à moitié, dans le textile (8).
